# EUROPEAN PATENT APPLICATION

(11) **EP 3 150 717 A1**
(43) Date of publication of application: **05.04.2017**
(21) Application number: 15799977.2
(22) Date of filing: 27.05.2015
(51) Int. Cl.: C12Q 1/68

(54) **PIK3CA GENE MUTATION DETECTION KIT**

(30) Priority: 29.05.2014 CN 201410232729
(71) Applicant: Beijing ACCB Biotech Ltd., Beijing 100094 (CN)
(72) Inventor: MO, Minli, Beijing 100094 (CN); LI, Hui, Beijing 100094 (CN); CHEN, Zhao, Beijing 100094 (CN); DING, Feng, Beijing 100094 (CN); LI, Jun, Beijing 100094 (CN)
(74) Representative: Tergau & Walkenhorst Patentanwälte - Rechtsanwälte
(86) International application number: PCT/CN2015/000362
(87) International publication number: WO 2015/180488

(57) **Abstract**

The present invention relates to a kit for detecting PIK3CA gene mutation, and this kit can be used to detect cancer-relatedPIK3CA gene mutation. The said kit comprises: (1) internal reference detection reagent, which includes internal reference gene specific primers, internal reference gene specific probes and dNTP solution; (2) PIK3CA mutation detection reagent, which includes PIK3CA mutant gene specific primers, PIK3CA mutant gene specific probes, internal control gene specific primers, internal control gene specific probes and dNTP solution; (3) Taq DNA polymerase; and (4) PIK3CA positive quality control.

Sequence List

<110>  Beijing ACCB Biotech Ltd.

<120>  Kit for detecting PIK3CA gene mutation

<130>  ykb012

<160>  19

<170>  PatentIn version 3.3

<210>  1
<211>  20
<212>  DNA
<213>  artificial

<220>
<223>  internal reference gene specific primer (forward primer, IR-F)

<400>  1
cagatgtgga tcagcaagca                                                    20


<210>  2
<211>  21
<212>  DNA
<213>  artificial

<220>
<223>  internal reference gene specific primer (reverse primer, IR-R)

<400>  2
catagtccgc ctagaagcat t                                                  21


<210>  3
<211>  19
<212>  DNA
<213>  artificial

<220>
<223>  internal control gene specific primer (forward primer, IC-F)

<400>  3
gatcagcaag caggagtat                                                     19


<210>  4
<211>  19
<212>  DNA
<213>  artificial

<220>
<223>  internal control gene specific primer (reverse primer, IC-R)

<400>  4
ggtgtaacgc aactaagtc                                                     19


<210>  5
<211>  21
<212>  DNA

<213>  artificial

<220>
<223>  PIK3CA mutant gene primer (PM1 forward primer, PM1-F)

<400>  5
ctacacgaga tcctctctca a                                                21


<210>  6
<211>  21
<212>  DNA
<213>  artificial

<220>
<223>  PIK3CA mutant gene primer (PM2 forward primer, PM2-F)

<400>  6
atcctctctc tgaaatcaca a                                                21


<210>  7
<211>  21
<212>  DNA
<213>  artificial

<220>
<223>  PIK3CA mutant gene primer (PM3 forward primer, PM3-F)

<400>  7
cctctctctg aaatcactgt t                                                21


<210>  8
<211>  21
<212>  DNA
<213>  artificial

<220>
<223>  PIK3CA mutant gene primers (PM1, PM2, PM3 reverse primers, PM123-R

<400>  8
aagaaacaga gaatctccat t                                                21


<210>  9
<211>  20
<212>  DNA
<213>  artificial

<220>
<223>  PIK3CA mutant gene primer (PM4 forward primer, PM4-F)

<400>  9
aaacaaatga atgatgcagg                                                  20


<210>  10
<211>  21
<212>  DNA
<213>  artificial

<220>

<223>  PIK3CA mutant gene primer (PM5 forward primer, PM5-F)

<400>  10
gaaacaaatg aatgatgcag t                                                21


<210>  11
<211>  20
<212>  DNA
<213>  artificial

<220>
<223>  PIK3CA mutant gene primers (PM4, PM5 reverse primers, PM45-R)

<400>  11
ttcagttcaa tgcatgctgt                                                  20


<210>  12
<211>  23
<212>  DNA
<213>  artificial

<220>
<223>  PIK3CA mutant gene probes (PM1, PM2, PM3 probes, PM123-pb)

<400>  12
agcacttacc tgtgactcca tag                                              23


<210>  13
<211>  21
<212>  DNA
<213>  artificial

<220>
<223>  PIK3CA mutant gene probes (PM4, PM5 probes, PM45-pb)

<400>  13
tttgttgtcc agccaccatg a                                                21


<210>  14
<211>  23
<212>  DNA
<213>  artificial

<220>
<223>  internal reference gene specific probe (IR-pb)£©

<400>  14
atgacgagtc cggcccctcc atc                                              23


<210>  15
<211>  22
<212>  DNA
<213>  artificial

<220>
<223>  internal control gene specific probe (IC-pb)

<400>  15

tagtccgcct agaagcattt gc                                                22

<210>  16
<211>  79
<212>  DNA
<213>  unknown

<220>
<223>  amplified fragment of internal reference gene (IR)

<400>  16
cagatgtgga tcagcaagca ggagtatgac gagtccggcc cctccatcgt ccaccgcaaa      60

tgcttctagg cggactatg                                                   79

<210>  17
<211>  89
<212>  DNA
<213>  unknown

<220>
<223>  amplified fragment of internal control gene (IC)

<400>  17
gatcagcaag caggagtatg acgagtccgg cccctccatc gtccaccgca aatgcttcta      60

ggcggactat gacttagttg cgttacacc                                        89

<210>  18
<211>  93
<212>  DNA
<213>  unknown

<220>
<223>  amplified fragment of PIK3CA (containing Codon 542, 545)

<400>  18
ctacacgaga tcctctctct gaaatcactg agcaggagaa agattttcta tggagtcaca      60

ggtaagtgct aaaatggaga ttctctgttt ctt                                   93

<210>  19
<211>  89
<212>  DNA
<213>  unknown

<220>
<223>  amplified fragment of PIK3CA (containing Codon 1047)

<400>  19
gaaacaaatg aatgatgcac atcatggtgg ctggacaaca aaaatggatt ggatcttcca      60

cacaattaaa cagcatgcat tgaactgaa                                        89

## Description

### Technical field

The present invention relates to gene mutation detection. Specifically, the present invention relates to a kit for detecting PIK3CA gene mutation, and this kit can be used to detect cancer-related PIK3CA gene mutation.

### Background of the invention

In recent years, the morbidity of malignant tumor has been on a rising trend in China, with over 1.7 million new patients every year. Lung cancer is one of the most common malignant tumors, with mortality ranking at the top among all malignant tumors, and the morbidity of non-small cell lung cancer (NSCLC) is the highest, accounting for about 80% of all lung cancer patients. Colorectal cancer is a malignant tumor of digestive tract, with morbidity ranking at the fourth place among common tumors. Breast cancer is a common malignant tumor of women. In China, there are about 180,000 new breast cancer patients every year.

The Food and Drug Administration (FDA) has approved to use Gefitinib (trade name Iressa) and Erlotinib (trade name Tarceva), which are TKI drugs targetinghuman epidermal growth factor receptor (EGFR) tyrosine kinase, in the treatment of non-small cell lung cancer; use Cetuximab (trade name Erbitux) and Panitumumab (trade name Vectibix), which targethuman epidermal growth factor receptor (EGFR), in the treatment of colorectal cancer; and use Trastuzumab (trade name Herceptin), which targetshuman epidermal growth factor receptor 2 (HER2), in the treatment of breast cancer. As the theory and practices of gene detection before the selection of targeted drugs for tumor patients have become mature in European countries and USA, the Clinical Therapy Steering Committee of China has also gradually included the gene detection into the treatment guidelines of relevant diseases. It has been found in researches that, the mutations of PIK3CA gene Exons 9 and 20 are related to the resistance to TKI drugs and monoclonal antibody drugs targeting EGFR or HER2; drugs targetingPIK3CA carrying mutations are also under research^{[1, 2]}.

The catalytic subunit (subunit alpha) oftype IA phosphatidylinositol-4,5-bisphosphate 3-kinase (PIK3CA)encodes type I phosphatidylinositol-3-kinase, positioned at 3q26, with a length of 34 kb, containing 20 exons, and encoding 1068 amino acids, and this group of amino acids produce a group of protein with a length of 124kD. Under physiological conditions, the PIK3CAgene is expressed in the normal brain, lung, breast, stomach and intestine, cervix, ovary etc., with many important physiological functions such as regulating the proliferation, differentiation and living of somatic cells, but mostly existing in the non-activated form, and usually not easily detectable; after its mutation, both the gene and its protein can overexpress and can be detected^{[3]}. Researches have shown that, the mutation of PIK3CA gene can not only enhance the catalytic activity of PI3Ks, but also promote canceration of cells^{[4]}. At present, PIK3CA has already been proved as an oncogene, and its mutation includes the gene amplification, deficiency, missense mutation of somatic cellularity and so on. It has been found in the research that mutation of oncogene PIK3CA exists in about 30% of human solid tumors, and the mutation proportion in the colon cancer, glioblastoma, stomach cancer, breast cancer and lung cancer is respectively about 32%, 27%, 25%, 25% and 4%.However, PIK3CA mutation is rarely seen in the tumors in the biliary system and in diffused large B cell lymphoma^{[5]}.

About 4/5 of PIK3CA mutations take place at the two hot spot areas of Exon 9 (E542K, E545D, E545K mutation sites) and Exon 20 (H1047R, H1047L). These mutations can not only reduce cell apoptosis, but also promote the tumor infiltration and increase the activity of its downstream kinase PI3Ks. Researches about these two hot spot areas of PIK3CA mutation have revealed that, mutations in the kinase zone and spiral zone can lead to change of enzyme functionality through different mechanisms. Mutations in different zones lead to activation of PI3Ks respectively through the different mechanisms of interaction with PI3Ks regulatory subunit p85 and RAS-GTP^{[6]}.

In addition, it has been found in the research for all mutation sites that, all cells containing PIK3CA mutations have high enzymatic activity and conversion capacity. All these results show that cells with PIK3CA mutations have changed in their molecular biological function and cellular biological features. PIK3CA mutations can regulate the living, proliferation and adherency processes of cells by upregulating the PI3K activity and further activatingits downstream molecules such as AKT. It is believed that PIK3CA mutation can possibly participate in the process of regulating the canceration of cells^{[7]}.

It has been found in the research that carrying PIK3CA mutation may lead to gefitinib resistance in the lung cancer patients^{[8]}, lead to trastuzumab resistance in the breast cancer patients^{[9-11]}, and lead to cetuximab and panitumumabresistance in the colorectal cancer patients ^{[12-14]}.

### Summary of the invention

In the present invention, the kit for detecting has been designed for the following detection sites:

**Table 1. Mutation detection sites**

| code | gene | mutation position | mutated base | mutated amino acid |
|---|---|---|---|---|
| | Internal reference gene ACTB | | | |
| PM1 | PIK3CA | Exon 9 | 1624G>A | E542K |
| PM2 | PIK3CA | Exon 9 | 1633G>A | E545K |
| PM3 | PIK3CA | Exon 9 | 1635G>T | E545D |
| PM4 | PIK3CA | Exon 20 | 3140A>G | H1047R |
| PM5 | PIK3CA | Exon 20 | 3140A>T | H1047L |

Specifically, the present invention relates to a kit for detecting PIK3CA gene mutation, comprising:
(1) internal reference detection reagent, which includes the internal reference gene specific primers, internal reference gene specific probes and dNTP solution;
(2) PIK3CA mutation detection reagent, which includes the PIK3CA mutant gene specific primers, PIK3CA mutant gene specific probes, internal control gene specific primers, internal control gene specific probes and dNTP solution;
(3) Taq DNA polymerase; and
(4) PIK3CA positive quality control, which includes the IR gene, PIK3CA mutant gene and internal control gene fragments.

More specifically, in the above-mentioned kit, internal reference gene specific primers in the said internal reference detection reagent are SEQ ID NO: 1 and SEQ ID NO: 2; in the said internal reference detection reagent the internal reference gene specific probe is SEQ ID NO: 14; the PIK3CA mutant gene in the said PIK3CA mutation detection reagent is selected from: PM1, i.e. PIK3CA gene Exon 9 1624G>A; PM2, i.e. PIK3CA gene Exon 9 1633G>A; PM3, i.e. PIK3CA gene Exon 9 1635G>T; PM4, i.e. PIK3CA gene Exon 203140A>G; and PM5, i.e. PIK3CA gene Exon 203140A>T. The kit of the present invention can be used to detect at least one mutations of PM1, PM2, PM3, PM4 and PM5.

In the said PIK3CA mutation detection reagent, the PIK3CA mutant gene specific primers are as follows: for PM1 mutation the primers are SEQ ID NO:5 and SEQ ID NO: 8; for PM2 mutation the primers are SEQ ID NO:6 and SEQ ID NO: 8; for PM3 mutation the primers are SEQ ID NO:7 and SEQ ID NO: 8; for PM4 mutation the primers are SEQ ID NO:9 and SEQ ID NO: 11; for PM5 mutation the primers are SEQ ID NO:10 and SEQ ID NO: 11; among them, in the said PIK3CA mutation detection reagent the PIK3CA mutant gene specific probes are selected from SEQ ID NO: 12 or SEQ ID NO: 13; in the said PIK3CA mutation detection reagent the internal control gene specific primers are SEQ ID NO: 3 and SEQ ID NO: 4; in the said PIK3CA mutation detection reagent the internal control gene specific probe is SEQ ID NO: 15; the 5' end of the said probe is connected with the FAM radical, and the 3' end connected with the BHQ1 radical; the end concentration of the said dNTP solution is 400µM; the said internal reference gene sequence is SEQ ID No: 16; the said internal control gene sequence is SEQ ID No: 17; and the sequence of said PIK3CA gene is SEQ ID No: 18 or SEQ ID No: 19.

Using the kit of the present invention in the PIK3CA gene mutation detection for lung cancer, breast cancer, colorectal cancer patients enables accurate prediction of the effectiveness of the corresponding targeted drugs (such as gefitinib, trastuzumab, cetuximab etc.), so as to facilitate clinical selection of drugs, effectively improve treatment results and provide the maximum benefit to patients; in the meanwhile, it can also avoid medical expense burden on patients and waste of public medical resources resulted from unreasonable application of drugs, and reduce unnecessary loss of time and money.

### Brief description of the drawings

Fig. 1 shows the result of PM1 mutation detection, in which the content of the wild type genomeDNA is 20ng/µl, the content of mutant genome DNA is 10ng/µl, and respectively contains 10, 5, 1,0.5% mutation.
Fig. 2 shows the result of PM2 mutation detection, in which the content of the wild type genome DNA is 20ng/µl, the content of mutant genome DNA is 10ng/µl, and respectively contains 10, 5, 1,0.5% mutation.

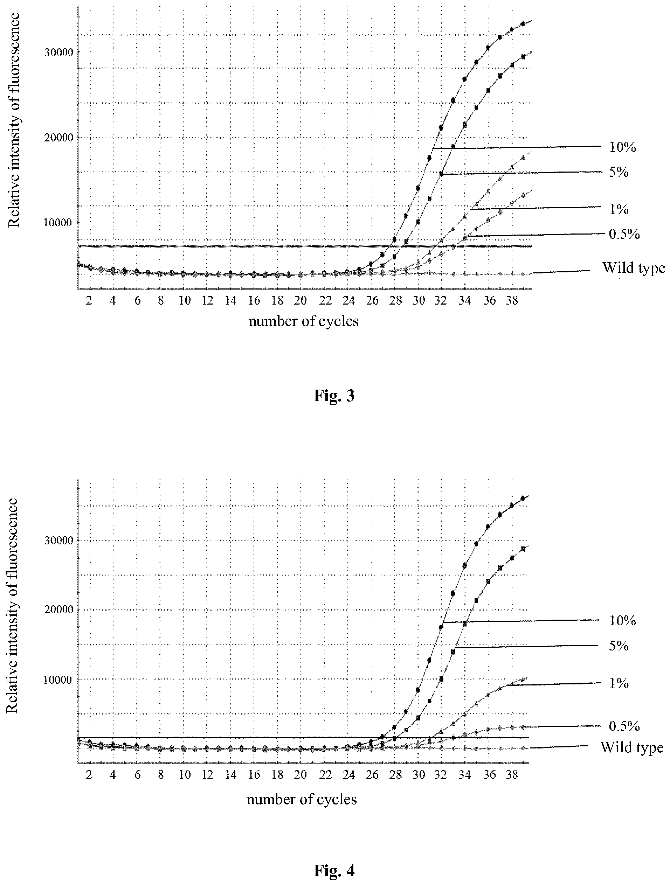

Fig. 3 shows the result of PM3 mutation detection, in which the content of the wild type genome DNA is 20ng/µl, the content of mutant genome DNA is 10ng/µl, and respectively contains 10, 5, 1, 0.5% mutation.
Fig. 4 shows the result of PM4 mutation detection, in which the content of the wild type genome DNA is 20ng/µl, the content of mutant genome DNA is 10ng/µl, and respectively contains 10, 5, 1, 0.5% mutation.

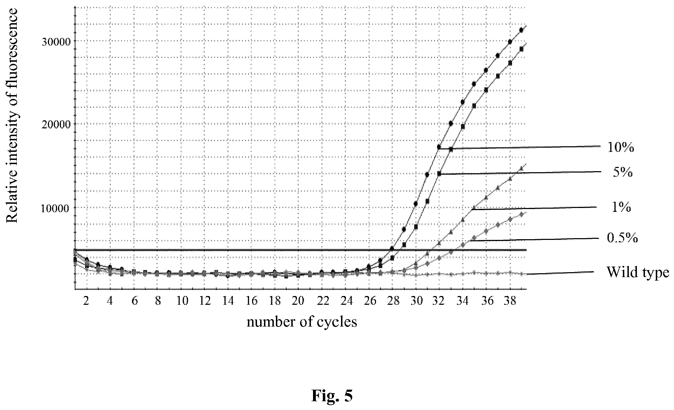

Fig. 5 shows the result of PM5 mutation detection, in which the content of the wild type genome DNA is 20ng/µl, the content of mutant genome DNA is 10ng/µl, and respectively contains 10, 5, 1, 0.5% mutation.

### Examples

### 1. Experiment method

The real-time fluorescent PCR technology was adopted. The ARMS (amplification refractory mutation system) method was used to detect gene mutation. That is, gene mutation was detected by using primers 3' end to identify mutation, in conjunction with the TaqMan probe hydrolysis luminescence.

The kit includes the internal reference (IR) detection and internal control (IC) detection. The IR gene is a housekeeping gene different from the PIK3CA geneto be tested. By detecting the amplification of the IR gene (FAM channel), analysis can be made whether the DNA to be tested can be normally amplified, so as to exclude the possible causes of PCR detection failure such as poor DNA purity and concentration, or containing PCR inhibitor. In this kit, the IC detection system is also provided in the detection systems for various mutation types of PIK3CA gene. The two systems react in the same PCR tube at the same time. The IC gene is also a housekeeping gene different from the PIK3CA gene to be tested. The probe identifying the PIK3CA gene mutation template was modified with a FAM fluorescent radical, and the probe identifying the IC gene template was modified with the HEX fluorescent radical. By detecting the amplification of the IC gene (HEX channel), analysis can be made whether the DNA to be tested can be normally amplified, so as to exclude the possible causes of PCR detection failure such as missing reagent or specimen, or specimen containing PCR inhibitor.

### 2. Composition of kit (Table 2):

| name | components |
|---|---|
| IR detection reagent | including the internal reference gene (housekeeping gene different from the PIK3CA gene to be tested) specific primers, probe and dNTP solution; |
| PM1, PM2, PM3, PM4, PM5 detection reagent | including PIK3CA gene mutant and internal control gene specific primers, probes and dNTP solution |
| Taq DNA polymerase | Taq DNA polymerase |
| PIK3CA positive quality control (PC) | including internal reference gene, PIK3CA gene mutant and internal control gene fragment |

### 2.1 IR, PM1∼5 detection reagent, Taq DNA polymerase (Table 3):

| name of raw material | source of raw material | end concentration in the detection system |
|---|---|---|
| Taq enzyme buffer | Tiangen | 1× |
| Magnesium chloride | Tiangen | 3 mM |
| dNTP (containing dATP, dTTP, dCTP, dGTP) | Tiangen | 400 µM |
| Forward primer | Shenggong | 500 nM |
| Reverse primer | Shenggong | 500 nM |
| Probe | Shenggong | 300 nM |
| Taq DNA polymerase | Tiangen | 0.05 U/µl |

### 2.2 positive quality control (PC):

Artificially cloned on the pMD18T plasmid.

### 2.3 Primer and probe sequences (Table 4):

| classification | name | sequence (5'-3') | SEQ ID NO |
|---|---|---|---|
| primers | IR-F | CAGATGTGGATCAGCAAGCA | 1 |
| | IR-R | CATAGTCCGCCTAGAAGCATT | 2 |
| | IC-F | GATCAGCAAGCAGGAGTAT | 3 |
| | IC-R | GGTGTAACGCAACTAAGTC | 4 |
| | PM1-F | CTACACGAGATCCTCTCTCAA | 5 |
| | PM2-F | ATCCTCTCTCTGAAATCACAA | 6 |
| | PM3-F | CCTCTCTCTGAAATCACTGTT | 7 |
| | PM123-R | AAGAAACAGAGAATCTCCATT | 8 |
| | PM4-F | AAACAAATGAATGATGCAGG | 9 |
| | PM5-F | GAAACAAATGAATGATGCAGT | 10 |
| | PM45-R | TTCAGTTCAATGCATGCTGT | 11 |
| probes | PM123-pb | AGCACTTACCTGTGACTCCATAG (5'-FAM, 3'-BHQ1) | 12 |
| | PM45-pb | TTTGTTGTCCAGCCACCATGA (5'-FAM, 3'-BHQ1) | 13 |
| | IR-pb | ATGACGAGTCCGGCCCCTCCATC (5'-FAM, 3'-BHQ1) | 14 |
| | IC-pb | TAGTCCGCCTAGAAGCATTTGC (5'-HEX, 3'-BHQ1) | 15 |

The internal reference gene and internal control gene fragments in PIK3CA positive quality control (PC) can be obtained from for example the NCBI nucleotide database (http://www.ncbi.nlm.nih.gov/nuccore). For example, through the accession No. NG_007992.1, we can obtain the amplified fragment of internal reference gene (IR): 
CAGATGTGGATCAGCAAGCAGGAGTATGACGAGTCCGGCCCCTCCATCGTCCACC
GCAAATGCTTCTAGGCGGACTATG (SEQ ID No: 16)
 and the amplified fragment of internal control gene (IC)

GATCAGCAAGCAGGAGTATGACGAGTCCGGCCCCTCCATCGTCCACCGCAAATGC
TTCTAGGCGGACTATGACTTAGTTGCGTTACACC (SEQ ID No: 17)。

From this database, we can also obtain the amplified fragment of PIK3CA containing Codon 542 and 545:

CTACACGAGATCCTCTCTCTGAAATCACTGAGCAGGAGAAAGATTTCTATGGA
GTCACAGGTAAGTGCTAAAATGGAGATTCTCTGTTTCTT (SEQ ID No: 18)

Condon 542 and 545 are shown in boxes.

From this database, we can also obtain the amplified fragment of PIK3CA containing Condon 1047:

GAAACAAATGAATGATGCACATCATGGTGGCTGGACAACAAAAATGGATTGGAT
CTTCCACACAATTAAACAGCATGCATTGAACTGAA (SEQ ID No: 19)

Codon 1047 is shown in box.

### 3. Embodiments

### 3.1 Specificity and sensitivity:

We detected 20ng/µl genome DNA containingwild typePIK3CA only, and 10ng/µl genome DNA containing 10, 5, 1, 0.5% PIK3CA mutation respectively (mutation percentage = mutant type/wild type× 100%).

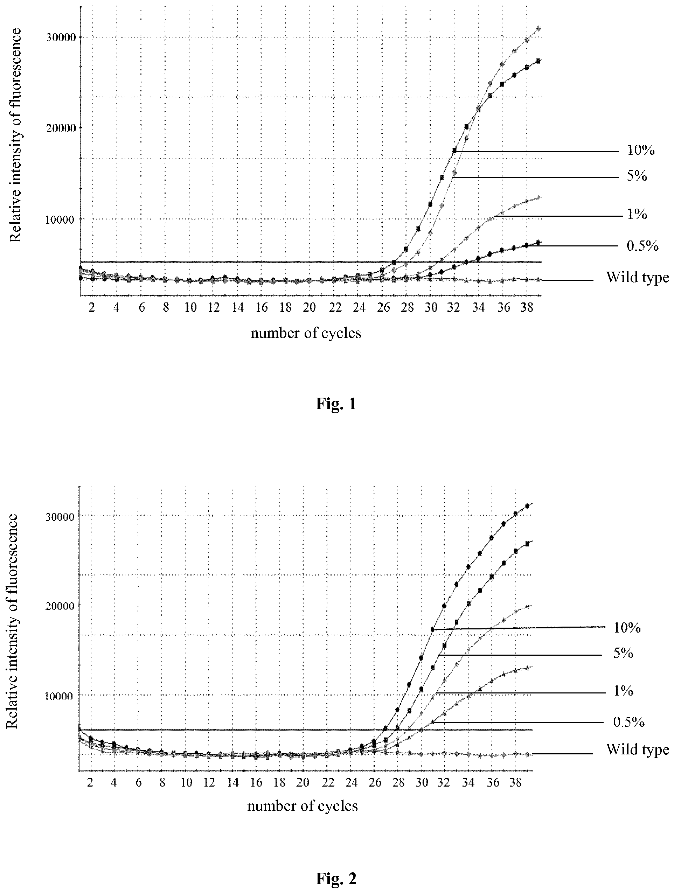

Attached figures 1∼5 respectively show the mutation detection results for PM1∼5.

It can be seen from the results shown in attached figures 1∼5 that:
(1) The kit of the present invention has good specificity. The kit of the present invention is used to detect the mutant type DNA. The figures show that there is no amplification signal after adding the wild type DNA (in the figures, the lines indicated with "wild type" are basically horizontal), indicating good specificity of the detection reagent and it will not produce "false positive result". Here, "false positive result" means the amplification curve appears after adding the wild type DNA.
(2) The kit of the present invention has good sensitivity. Amplification can be demonstrated when mutant type DNA is added. And it can detect 10 ng/µl wild type genome DNA containing 10, 5, 1 and 0.5% mutation, showing "good sensitivity". The curves in figures indicated with different percentages shows the details.

### 3.2 Comparison of methods (the kit of the present invention and the Sanger sequencing method):

**Table 5. The kit of the present invention and the test result of Sanger sequencing method with four-fold table method**

| | | Sanger sequencing method | |
|---|---|---|---|
| | | positive (mutant type) | negative (wild type) |
| kit of the present invention | positive (mutant type) | 35 | 3 |
| | negative (wild type) | 0 | 262 |

It includes 100 cases each for lung cancer, breast cancer and colorectal cancer.

The four-fold table method is a means to analyze the Receiver Operating Characteristic (ROC) which is familiar to the technical person in this art.

If the Sanger sequencing method is taken as the "gold standard" for testing gene mutation, then it can be obtained by calculation from Table 5 that: the clinical sensitivity of PIK3CA kit = 35/(35+0)=100%, its clinical specificity = 262/(3+262)=98.9%, and overall consistency = (35+262)/(35+262+3+0) =99.0%. This shows that the kit of the present invention has very high clinical sensitivity, clinical specificity and overall consistency.

In addition, it can be seen from the data of the four-fold table that, all specimens shown positive with Sanger sequencing method are positive when detected with quantitative PCR (QPCR), but specimens detected as positive with QPCR can be detected as negative with the Sanger sequencing method, indicating that the QPCR kit of the present invention has a higher sensitivity than that of the Sanger sequencing method.

### References

[1] Gustin JP, Cosgrove DP, Park BH. The PIK3CA gene as a mutated target for cancer therapy[J]. Curr Cancer Drug Targets, 2008,8(8): 733-740.
[2] Janku F, Wheler JJ, Naing A, et al. PIK3CA mutation H1047R is associated with response to PI3K/AKT/mTOR signaling pathway inhibitors in early-phase clinical trials[J]. Cancer Res, 2013,73(1): 276-284.
[3] Volinia S, Hiles I, Ormondroyd E, et al. Molecular cloning, cDNA sequence, and chromosomal localization of the human phosphatidylinositol 3-kinase p110 alpha (PIK3CA) gene[J]. Genomics, 1994,24(3): 472-477.
[4] Oxnard GR, Binder A, Janne PA. New targetable oncogenes in non-small-cell lung cancer[J]. J Clin Oncol, 2013,31(8): 1097-1104.
[5] Samuels Y, Waldman T. Oncogenic mutations of PIK3CA in human cancers[J]. Curr Top Microbiol Immunol, 2010,347: 21-41.
[6] Schildgen V, Lusebrink J, Appel JD, et al. Identification of uncommon PIK3CA mutations in lung cancer by using pyrosequencing[J]. Diagn Mol Pathol, 2013,22(1): 22-27.
[7] Chaft JE, Arcila ME, Paik PK, et al. Coexistence of PIK3CA and other oncogene mutations in lung adenocarcinoma-rationale for comprehensive mutation profiling[J]. Mol Cancer Ther, 2012,11(2): 485-491.
[8] Ludovini V, Bianconi F, Pistola L, et al. Phosphoinositide-3-kinase catalytic alpha and KRAS mutations are important predictors of resistance to therapy with epidermal growth factor receptor tyrosine kinase inhibitors in patients with advanced non-small cell lung cancer[J]. J Thorac Oncol, 2011,6(4): 707-715.
[9] Berns K, Horlings HM, Hennessy BT, et al. A functional genetic approach identifies the PI3K pathway as a major determinant of trastuzumab resistance in breast cancer[J]. Cancer Cell, 2007,12(4): 395-402.
[10] Chandarlapaty S, Sakr RA, Giri D, et al. Frequent mutational activation of the PI3K-AKT pathway in trastuzumab-resistant breast cancer[J]. Clin Cancer Res, 2012,18(24): 6784-6791.
[11]Pohlmann PR, Mayer IA, Mernaugh R. Resistance to Trastuzumab in Breast Cancer[J]. Clin Cancer Res, 2009,15(24): 7479-7491.
[12] Sartore-Bianchi A, Martini M, Molinari F, et al. PIK3CA mutations in colorectal cancer are associated with clinical resistance to EGFR-targeted monoclonal antibodies[J]. Cancer Res, 2009,69(5): 1851-1857.
[13] Bardelli A, Siena S. Molecular mechanisms of resistance to cetuximab and panitumumab in colorectal cancer[J]. J Clin Oncol, 2010,28(7): 1254-1261.
[14] Mao C, Yang ZY, Hu XF, et al. PIK3CA exon 20 mutations as a potential biomarker for resistance to anti-EGFR monoclonal antibodies in KRAS wild-type metastatic colorectal cancer: a systematic review and meta-analysis[J]. Ann Oncol, 2012,23(6): 1518-1525.

## Claims

1. A kit for detecting PIK3CA gene mutation, comprising:
(1) internal reference detection reagent, which includes the internal reference gene specific primers, internal reference gene specific probes and dNTP solution,
in which the said internal reference gene specific primers are SEQ ID No: 1 and SEQ ID No: 2;
the said internal reference gene specific probe is SEQ ID No: 14;
(2) PIK3CA mutation detection reagent, which includes PIK3CA mutant gene specific primers, PIK3CA mutant gene specific probes, internal control gene specific primers, internal control gene specific probes and dNTP solution,
in which the said PIK3CA mutant gene is selected from:
PM1, i.e. PIK3CA gene Exon 9 1624G>A;
PM2, i.e. PIK3CA gene Exon 9 1633G>A;
PM3, i.e. PIK3CA gene Exon 9 1635G>T;
PM4, i.e. PIK3CA gene Exon 20 3140A>G; and
PM5, i.e. PIK3CA gene Exon 20 3140A>T;
for PM1 mutation, the said PIK3CA mutant gene specific primers are SEQ ID NO: 5 and SEQ ID NO: 8;
for PM2 mutation, the said PIK3CA mutant gene specific primers are SEQ ID NO: 6 and SEQ ID NO: 8;
for PM3 mutation, the said PIK3CA mutant gene specific primers are SEQ ID NO: 7 and SEQ ID NO: 8;
for PM4 mutation, the said PIK3CA mutant gene specific primers are SEQ ID NO: 9 and SEQ ID NO: 11;
for PM5 mutation, the said PIK3CA mutant gene specific primers are SEQ ID NO: 10 and SEQ ID NO: 11;
the said PIK3CA mutant gene specific probe is SEQ ID No: 12 or SEQ ID No: 13;
the said internal control gene specific primers are SEQ ID No: 3 and SEQ ID No: 4;
the said internal control gene specific probe is SEQ ID No: 15;
(3) Taq DNA polymerase; and
(4) PIK3CA positive quality control, which includes internal reference gene, PIK3CA mutant gene and internal control gene fragments;
in which the said internal reference gene sequence is SEQ ID No: 16;
the said internal control gene fragment sequence is SEQ ID No: 17;
the sequence of said PIK3CA geneis SEQ ID No: 18 or SEQ ID No: 19.

2. The kit according to Claim 1, in which the 5' end of the said probe is connected with FAM, and the 3' end is connected with BHQ1.

3. The kit according to Claim 1, in which the end concentration of the said dNTP solution is 400µM.
